# EUROPEAN PATENT SPECIFICATION

(11) **EP 1 866 148 B1**
(45) Date of publication and mention of the grant of the patent: **11.05.2011**
(21) Application number: 06744443.0
(22) Date of filing: 20.03.2006
(51) Int. Cl.: B32B 5/24, B32B 9/04, B32B 27/40, A61K 9/70, A61L 15/28, A61L 15/42, A61L 15/44, A61F 13/02, A41D 31/02, A61K 8/02

(54) **MULTI-PLY TECHNICAL COMPOSITE WHICH IS BREATHABLE AND MOISTURISING, RELEASES ACTIVE CONSTITUENTS AND PROMOTES THEIR ABSORPTION, AND CAN BE USED AS A MEDICAL AID OR TO MAKE CLOTHING OR STRUCTURES THAT COME INTO DIRECT CONTACT WITH HEALTHY OR DISEASED SKIN**
ATMUNGSFÄHIGER UND FEUCHTIGKEITSSPENDENDER MEHRLAGIGER TECHNISCHER VERBUNDWERKSTOFF, DER AKTIVE BESTANDTEILE ABGIBT UND IHRE ABSORPTION FÖRDERT UND ALS MEDIZINISCHE HILFE ODER ZUR HERSTELLUNG VON BEKLEIDUNG ODER STRUKTUREN VERWENDET WERDEN KANN, DIE IN DIREKTEN KONTAKT MIT GESUNDER ODER ERKRANKTER HAUT KOMMEN
COMPOSITE TECHNIQUE MULTICOUCHE LAISSANT PASSER L'AIR ET L'HUMIDITE, LIBERANT DES COMPOSANTS ACTIFS ET FAVORISANT LEUR ABSORPTION, UTILISABLE COMME ACCESSOIRE MEDICAL OU POUR LA CONFECTION DE VETEMENTS OU DE STRUCTURES ENTRANT EN CONTACT DIRECT AVEC LA PEAU SAINE OU MALADE

(30) Priority: 22.03.2005 IT MI20050467
(43) Date of publication of application: 19.12.2007
(73) Proprietor: Bottini, Emilio, 21019 Somma Lombardo (IT)
(72) Inventor: Bottini, Emilio, 21019 Somma Lombardo (IT)
(74) Representative: Minoja, Fabrizio
(86) International application number: PCT/IB2006/000616
(87) International publication number: WO 2006/100561

(56) References cited:
- EP-A- 0 099 758
- EP-A- 0 541 390
- EP-A- 1 088 535
- EP-A- 1 118 705
- EP-A- 1 132 084
- WO-A-03/092756
- DE-A1- 4 003 764
- GB-A- 1 493 823
- US-A- 5 759 570
- US-A- 5 823 983
- US-A1- 2002 099 318

## Description

This invention relates to a multi-ply technical composite which is breathable and moisturising, releases active constituents and promotes their absorption, and can be used in particular as a medical device or to make clothing or structures that come into direct contact with healthy or diseased skin.

### Background to the invention

The textile industry is particularly interested in the needs of the market; demand for materials dedicated to specific functions and, in the case of textile fibres and fabrics, functions which can interact with both healthy and diseased skin, has increased, especially in recent years.

The materials currently used have not proved entirely satisfactory.

### Description of the invention

The purpose of the invention is to provide a multi-ply technical composite which guarantees a given degree of breathability, so as to ensure optimum moisturisation of the skin and the ideal conditions for the operation of the active constituents.

Another purpose of the invention is to provide such a multi-ply composite which is usable in association with or incorporates cosmetic or pharmaceutical formulations, aiding their absorption and optimising their properties.

Yet another purpose of this invention is to provide a multi-ply composite which is flexible, comfortable and possibly antibacterial, and which can be recharged with the active constituent.

These aims are achieved, according to the invention, with the characteristics of the annexed independent claim 1.

The invention also relates to medical devices including the multi-ply composites described herein and their use as moisturising agents in dermatological and cosmetic applications.

Advantageous embodiments of the invention are expressed in the dependent claims.

### Detailed description of the invention

Substantially, the multi-ply technical composite according to the invention comprises at least two layers. The first of said layers, which comes into contact with the skin, is a fabric, and the second, external layer consists of a semipermeable membrane.

The first layer, which comes into contact with the skin, can be made of cotton (elasticised to a greater or lesser extent), silk, or fabrics to which chitosan, silver or another antibacterial substance or active constituent is added.

The second layer, constituted by a membrane of semipermeable material, is preferably made of polyurethane between 5 and 80 microns thick, with considerable elasticity in order to ensure the user's comfort. The membrane could be non-porous, so that the active constituent, if any, does not pass to the outside. In this way, the active constituent is not wasted, and as it does not reach the outer surface, it does not soil bedclothes, clothing or other dressings.

Said membrane is able to breathe by osmosis, and consequently does not form an occlusive dressing.

The annexed figure shows a schematic partial cross-section of a two-ply technical composite according to the invention, indicated globally as no. 1 and comprising a first inner layer 2 made of fabric, such as cotton, silk or another fibre, and a second outer layer 3 made of polyurethane, which are bonded firmly to one another, preferably by a hot-pressing process, using microdots of anallergic glue (4) specifically designed for this purpose, with a diameter which can be calculated at between 0.15 mm and 0.7 mm, placed at regular intervals, so that 60 to 180 dots per square centimetre can be counted. The regular spacing of the glue dots provides a suitable ratio between the glued surface and the free surface.

This structure optimises the inclusion of rechargeable receptors containing moisturising or more specifically therapeutic active constituents such as cyclodextrins, microsponges, liposomes and nanoparticles.

If applied to the skin (layer of cotton and/or other material in contact with the epidermis, and polyurethane membrane on the outside), depending on the objective parameters of intactness of the stratum corneum and the skin barrier, the multi-ply technical composite according to the invention improves the barrier and moisturising function of the skin; in other words, it acts as a moisturising substance. These properties can be demonstrated with the use of non-invasive methods such as transepidermal water loss (TEWL) and corneometry.

If used after the application of moisturising substances and/or active constituents, it promotes their activity and penetration.

The best results have been obtained with a composite of the type described above; in particular, a study has been conducted to evaluate the significant, innovative effects of the material on some skin physiology parameters.

The study was conducted on 15 subjects. The multi-ply composite was applied "as is" to the skin at two different sites: the volar surface of the forearm and the front abdominal region. The composite was left in position for eight consecutive hours to simulate the use of the product by consumers instead of underwear or pyjamas. Eight hours after the application, a number of instrumental tests were performed to investigate the changes which had taken place in the skin. In particular, the transepidermal water loss (TEWL) was measured, and the fluid content of the stratum corneum was measured by corneometry. The TEWL measurement measures the quantity of water vapour that evaporates on the surface of a given substrate, while corneometry is a technique based on detection of electrical capacitance which varies with the water content of a given body. All these tests were performed using a pure cotton fabric (placebo), applied by the same procedure, as control, and skin on which no product was positioned as negative control. The results demonstrated that after 8 hours' application of the test product to the skin surface, normal skin breathing through the membrane was maintained, with TEWL indexes comparable to those of cotton, associated with a (statistically significant) increased water content of the stratum corneum compared with cotton or the control skin.

This finding confirms the unique breathability of this product and its ability to increase skin moisturisation. Other membranes are unable to increase moisturisation when they are breathable. In a later study, which repeated the same test procedure, a moisturising cream was applied at the interface between product and skin. The result was even more marked (including statistically); high breathability was maintained (TEWL through the product), and associated with an excellent skin moisturising effect. This supports the use of the product in association with moisturising formulations in the treatment of various skin conditions characterised by dryness of the skin, such as diffuse xerosis, atopic dermatitis, psoriasis, etc.. In addition to the use of the product in association with cosmetic or parapharmaceutical formulations, materials used in the pharmaceutical and cosmetic industry for the controlled release of active constituents, such as cyclodextrins, microsponges, liposomes, nanoparticles and biopeptides, are incorporated in the finished product in order to increase the efficacy of said formulations.

It is known in skin pharmacology that the release and transcutaneous penetration of many active constituents takes place under optimum skin moisturising conditions; when the skin is dehydrated, these technologies often present problems with the linearity of controlled release, and therefore cannot be exploited to the full for therapeutic purposes. Some practical uses of the multi-ply composite according to the invention containing said slow-release particles (cyclodextrins, microspheres, liposomes etc.) could be their combination with active substances to treat blemishes caused by cellulite, topical anti-inflammatory substances (cortisone, NSAIDs, plant protection products, glycyrrhetinic acid, vitamins and antioxidant substances), or the treatment of desquamative dermatitis (urea, vitamin D derivatives, retinoids etc.).

Moreover, antiseptic and antibacterial active constituents nf various kinds such as chlorhexidine, triclosan, undecylenic acid, climbazole and other imidazole derivatives can be carried in controlled-release systems associated with the multi-ply composite and used for the treatment of impetiginous dermatitis, yeast infections and fungi in general, etc. The inclusion in the multi-ply system of silver (in various forms) also allows a preventive antimicrobial action to be performed to prevent and control bacterial and/or fungal infections in various disorders such as atopic dermatitis, various kinds of ulcerative lesions of the lower limbs, skin complications associated with some metabolic and inflammatory disorders such as diabetes, connective tissue disease and vascular diseases in general.

In particular it could be used in garments dedicated to the disorder or the part of the body to be treated, such as t-shirts, with or without sleeves, trousers, pyjamas, sheaths, gloves, caps, face masks, dressings, stockings, kneepads, underpants etc. for the treatment of various skin and other diseases, such as contact dermatitis, psoriasis, sprains and dislocations, joint problems, etc..

To sum up, the multi-ply technical composite according to the invention performs a breathing action by osmosis which allows sweat to be transported to the outside of the polyurethane membrane, thus preventing maceration, while it prolongs and optimises the action of any active or moisturising constituents (occlusive-like effect) so that the active constituent is not released to the exterior, in such a way that the outer surface of the membrane is not affected, and clothes, sheets, dressings and the like are not soiled, so that even patients with considerable problems do not need to interrupt their normal activities.

As already stated, this multi-ply composite can be used to make t-shirts, with or without sleeves, trousers, pyjamas, sheaths, gloves, caps, face masks and dressings.

A third outer layer with an aesthetic or finishing function can be bonded to this multi-ply technical fabric, again with glue dots or netting, using a hot-pressing process.

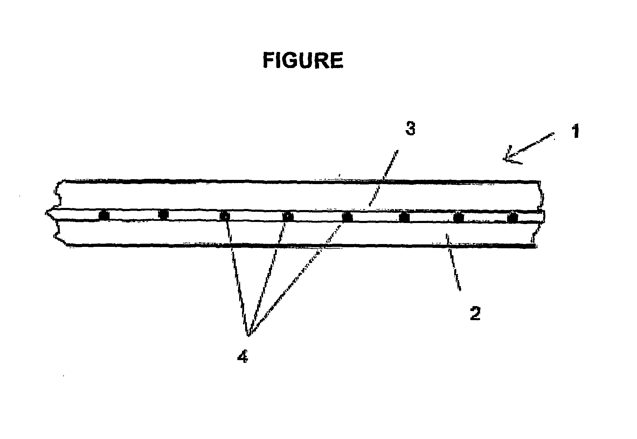

## Claims

1. Multi-ply composite particularly effective for medical aids and garments, comprising at least two layers, the first of which (2), in contact with the skin, is a fabric, while the second outer layer (3) is constituted by a semipermeable membrane, **characterised in that** the bonding of said semipermeable membrane forming the second, outer layer (3) to fabric layer (2) aids concentration and fixing of the receptors (cyclodextrins, nanoparticles, microsponges and biopeptides).

2. Multi-ply composite as claimed in claim 1, **characterised in that** it includes receptors selected from among cyclodextrins, microsponges, liposomes, nanoparticles and biopeptides, which can be recharged with moisturising or therapeutic active constituents.

3. Multi-ply composite as claimed in claim 2, used in combination with active substances in the treatment of blemishes caused by cellulite, topical anti-inflammatory substances, cortisone, NSAIDs, plant protection products, glycyrrhetinic acid, vitamins or antioxidants, or in desquamative dermatitis, such as urea, vitamin D derivatives and retinoids.

## Patentansprüche

1. Mehrschichtverbundstoff, welcher besonders geeignet ist für medizinissche Hilfsmittel und Kleidungsstücke, umfassend mindestens zwei Schichten, von denen die erste (2), in Kontakt mit der Haut, ein Gewebe ist, während die zweite, äußere Schicht (3) durch eine semipermeable Membran gebildet wird, **dadurch** gekenntzeichnet, dass die Bindung der semipermeablen Membran, die die zweite, äußere Schicht (3) bildet, an die Gewebeschicht (2) die Konzentration und Fixierung der Rezeptoren (Cyclodextrine, Nanoteilchen, Mikroschwämme und Biopeptide) unterstützt.

2. Mehrschichtverbundstoff nach Anspruch 1, **dadurch gekennzeichnet , dass** er Rezeptoren einschließt, die ausgewählt sind aus Cyclodextrinen, Mikroschwämmen, Liposomen, Nanoteilchen und Biopeptiden, welche wiederaufgeladen werden können mit befeuchtenden oder therapeutisch aktiven Bestandteilen.

3. Mehrschichtverbundstoff nach Anspruch 2, verwendet in Kombination mit aktiven Substanzen bei der Behandlung von Fehlstellen, welche verursacht werden durch Cellulite, topische anti-entzündliche Substanzen, Cortison, MSAIDs, Pflanzenschutzprodukte, Glycyrrhetinsäure, Vitamine oder Antioxidantien, oder bei schuppiger Dermatitis, wie zum Beispiel Harnstoff, Vitamin D Derivate und Retinoide.

## Revendications

1. Composite multi-épaisseurs particulièrement efficace pour des dispositifs d'appoint et des vêtements médicaux, comprenant au moins deux couches, la première d'entre elles (2), en contact avec la peau, est un textile, tandis que la seconde couche (3) extérieure est constituée d'une membrane semi-perméable, **caractérisé en ce que** la liaison de ladite membrane semi-perméable formant la seconde couche (3), extérieure à la couche textile (2) facilite la concentration et la fixation des récepteurs (cyclodextrines, nanoparticules, micro-éponges et biopeptides),

2. Composite multi-épaisseurs selon la revendication 1, **caractérisé en ce qu'**il comprend des récepteurs choisis parmi les cyclodextrines, les micro-éponges, les liposomes, les nanoparticules et les biopeptides, qui peuvent être rechargés par humidification ou par des constituants actifs sur le plan thérapeutique.

3. Composite multi-épaisseurs selon la revendication 2, utilisé en combinaison avec des substances actives dans le traitement des imperfections de la peau provoquées par la cellulite, des substances anti-inflammatoires topiques, la cortisone, des AINS, des produits de phytoprotection, l'acide glycirrhétinique, des vitamines ou antioxydants, ou dans la dermatite desquamative, tels que l'urée, les dérivés de vitamine D et les rétinoïdes.
